# FASCICULE DE BREVET EUROPEEN

(11) **EP 1 141 698 B1**
(45) Date de publication et mention de la délivrance du brevet: **28.02.2007**
(21) Numéro de dépôt: 00976111.5
(22) Date de dépôt: 07.11.2000
(51) Int. Cl.: G01N 31/22, G01N 33/50

(54) **PROCEDE POUR LA DETECTION DU MALONDIALDEHYDE (MDA)**
VERFAHREN ZUR BESTIMMUNG VON MALONDIALDEHYD (MDA)
METHOD FOR RAPIDLY DETERMINING THE PRESENCE OF MALONDIALDEHYDE (MDA) IN URINE, IN FOOD AND COSMETIC PRODUCTS

(30) Priorité: 09.11.1999 FR 9914070
(43) Date de publication de la demande: 10.10.2001
(73) Titulaire: Lauzanne, Eliane, 75010 Paris (FR); ROTHFUSS, Jacqueline, 67380 Lauterbourg (FR); COSPER SA, 92700 Colombes (FR)
(72) Inventeur: Morelle, Jean, decede (FR); Lauzanne, Eliane, 75011 Paris (FR); Rothfuss, Jaqueline, 67630 Lauterbourg (FR)
(86) Numéro de dépôt international: PCT/FR2000/003089
(87) Numéro de publication internationale: WO 2001/035091

(56) Documents cités:
- FR-A- 2 764 701
- GB-A- 2 045 429
- US-A- 4 318 706
- US-A- 4 367 285
- KIKUGAWA K.: "Use and limitation of thiobarbituric acid test for lipid peroxidaton " RECENT RES. DEVL. IN LIPIDS RES., vol. 1, 1997, pages 73-96, XP000937401
- HUANG T-S ET AL: "THE EFFECT OF FREE RADICALS ON HEPATIC 5' MONODEIODINATION OF THYROXINE AND 3 3' 5' TRIIODOTHYRONINE" ENDOCRINOLOGY, vol. 121, no. 2, 1987, pages 498-503, XP000937429 ISSN: 0013-7227

## Description

A la lumière de nombreux travaux,publiés dans le monde au cours de ces trente dernières années,il a été démontré que la dégradation oxydative des acides gras insaturés,qu'elle soit au niveau de la membrane cellulaire comme au niveau alimentaire, constitue un danger pour la santé.

Il est en effet connu,que la dégradation oxydative conduit à de multiples états pathologiques,que les lipoperoxydes se dégradent en générant diverses substances,formant des associations nouvelles avec les proteines,comme les bases de SCHIFF et les lipopigments.Parmi les substances les plus réactives,issues de cette dégradation,il faut citer le malondialéhyde (ou MDA) qui comporte 3 carbones et deux fonctions aldéhydiques de forte activité réactionnelle.

O=CH-CH₂-CH=O

On sait aujourd'hui,que le MDA urinaire est la réponse à l'intervention des radicaux libres;il est *un indice de peroxydation lipidique in vivo.* Il peut être la conséquence d'une alimentation comportant des lipoperoxydes,ou d'un dysfonctionnement métabolique,ou encore d'une déficience en antioxydants endogènes ou exogènes.

La recherche du MDA dans l'urine présente donc un intérêt considérable,elle permet de constater soit l'existence d'une alimentation oxydative dangereuse pour la santé,pouvant conduire à des perturbations biologiques exprimées par diverses pathologies (cardiovasculaires,cataracte,cancer etc..),soit de remonter jusqu'aux pathologies elles-mêmes.
Ce test permet de déterminer rapidement l'existence d'une perturbation métabolique,pouvant être liée soit à l'alimentation soit à un état pathologique.

La détermination du MDA urinaire,représente un élément important d'informations pour le medecin comme pour le nutritionniste.Or la recherche du MDA urinaire nécessite de coûteuses opérations analytiques,qui ne peuvent être effectuées qu'en laboratoire.

Ce nouveau procédé,simple,rapide et fiable,peut être utilisé par quiconque et permet entre deux et trois minutes,de mettre en évidence la présence de MDA aussi bien dans l'urine que dans des produits alimentaires ou des préparations cosmétiques. Il consiste simplement à déposer sur un papier réactif,une goutte d'urine ou de matière grasse pour mettre en évidence le MDA.

D'autre part,on pourra également,en ayant recours à des solutions étalons de MDA,réaliser une échelle à différentes concentrations pour connaître sa concentration urinaire par exemple.

Cette technique peut être utilisée pour la recherche du MDA dans les matières grasses,telle que les huiles utilisées pour l'alimentation.
Dans ce cas,du fait de la faible quantité de substance hydrophyle (MDA) dans une forte quantité d'huile lipophyle,on ajoutera un peu d'eau à l'huile,le mélange étant fortement agité pour obtenir une émulsion temporaire. On dépose sur le papier réactif une petite goutte de l'émulsion.La présence de MDA se manifeste par une couronne rouge se situant au centre de la diffusion de l'huile sur le papier.
On peut également laisser décanter la phase aqueuse qui sera prélevée,puis une goutte en sera déposée sur la papier test.On obtiendra une couronne caractéristique allant du rose au rouge mettant en évidence la présence de MDA. Si une certaine quantité d'huile est entraînée,on obtient un disque rougeâtre diffus.

On pourra également se servir de ce test pour contrôler l'absence de MDA dans différents corps gras et émulsions utilisés dans l'industrie cosmétique.

L'invention est caractérisée en ce qu'elle comporte un papier filtre,imprégné du réactif bien connu:l'acide thiobarbiturique, en presence d'un acide organique dont la capacité acide est voisine de celle de l'acide acétique,généralement employé anhydre pour solubiliser l'acide thiobarbiturique, à un pH se situant entre 1,0 et 3,0 et éviter ainsi les interférences. L'acide thiobarbiturique réagit avec le MDA pour donner un chromogène rouge.

Parmi les acides organiques expérimentés,l'acide glycolique a conduit à une grande sensibilité du test,permettant d'obtenir à l'endroit de la goutte versée,une couronne rouge trés caractéristique,alors que la couronne reste blanche quand l'urine est exempte de MDA.Les autres acides comme l'acide fumarique,l'acide citrique,l'acide tartrique ne permettent pas d'obtenir des couronnes nettes caractéristiques.

Dans le cadre de l'invention,on imprègne des bandes de papier filtre d'une solution contenant 0,5g % d'acide thiobarbiturique, 10g% d'acide glycolique,complétée à 100 ml d'eau distillée.Les bandes sont séchées à l'étuve vers 80°C et conservées à l'abri de l'air et de la lumière.

L'utilisation d'un tel test est peu onéreuse,et fiable,il suffit de présenter la bande comportant la goutte d'urine à une source de chaleur: étuve à 100°C,vapeur issue de l'ébullition de l'eau ou encore en approchant le papier d'une ampoule électrique.On voit apparaître en moins de trois minutes un cercle allant du rose au rouge,d'intensité variable selon la concentration en MDA du produit tésté. Dans le cas d'absence de MDA, le cercle est blanchâtre.

## Revendications

1. Procédé permettant de mettre rapidement en évidence le malondialdéhyde (MDA) dans l'urine, dans des produits alimentaires, et dans des produits cosmétiques, **caractérisé en ce qu'**on utilise une bande de papier filtre imprégnée d'acide thiobarbiturique solubilisé par une solution d'acide glycolique. On dépose sur ce papier réactif, soit une goutte d'urine, soit une goutte d'huile ou d'un extrait aqueux d'un produit alimentaire ou cosmétique, suivi d'une exposition à une source de chaleur, révélant un chromogène rouge issu de la réaction de l'acide thiobarbiturique avec le malondialdéhyde.

2. Procédé selon la revendication 1, **caractérisé en ce que** les bandes de papier filtre sont imprégnées d'une solution aqueuse contenant : 0,5 % d'acide thiobarbiturique solubilisé par 10g % d'acide glycolique à un pH se situant entre 1,0 et 3,0 séchées vers 80°C, et conservées à l'abri de l'air et de la lumière.

3. Procédé selon les revendications 1 et 2, **caractérisé en ce que** la présence de MDA urinaire est mise en évidence par la formation d'une couronne rouge dont l'intensité est liée à la concentration.

4. Procédé selon les revendications 1 et 2, **caractérisé en ce que** la présence de MDA dans les produits alimentaires ou cosmétiques est mise en évidence, soit par une couronne rouge se situant au centre de la diffusion de l'huile sur le papier, soit par une couronne caractéristique allant du rose au rouge, soit par un disque rougeâtre diffus.

## Claims

1. A procedure that rapidly demonstrates the presence of malondialdehyde (MDA) in urine, in food products and cosmetics. It is **characterized by** the use of a band of filter paper impregnated with thiobarbituric acid soluble in a solution of glycolic acid. The method consists of the placing of a small drop of urine, of an oil or aqueous extract of a food or cosmetic product on the reactive paper and subsequently exposing it to a heat source which will reveal a chromogenic red which is the result of the reaction of the thiobarbituric acid with the MDA.

2. The method described in Paragraph (1), is further **characterized by** the use of filter paper impregnated with an aqueous solution containing 0.5% of thiobarbituric acid made soluble by 10 grams of glycolic acid with a pH between 1.0 and 3.0, dried at around 80°C, and preserved without air and light.

3. Per the descriptions of the methods outlined in Paragraphs (1) and (2), the presence of MDA is indicated by the appearance of a red ring, whose intensity is directly linked to the quantity of MDA present in the substance being tested.

4. Per the descriptions of the methods outlined in Paragraphs (1) and (2), the presence of MDA in food or cosmetic products would be indicated either by a red ring forming in the center of the oil diffused on the filter paper, or by the characteristic ring which could tend from pink to red, or by a diffuse reddish disk.

## Patentansprüche

1. Verfahren zum schnellen Nachweis von Malondialdehyd (MDA) im Urin, in Lebensmittelprodukten und kosmetischen Produkten, **dadurch gekennzeichnet, dass** ein Streifen Filterpapier verwendet wird, der mit in einer Glykolsäure-Lösung solubilisierten Thiobarbitursäure getränkt wurde. Auf dieses Indikatorpapier wird entweder ein Tropfen Urin, oder ein Tropfen Öl, oder ein Tropfen eines wässrigen Auszugs aus einem Lebensmittel- oder Kosmetik-Produkt aufgetragen, und anschließend einer Wärmequelle ausgesetzt, was zu einer chromogenen Rotfärbung führt, hervorgerufen durch die Reaktion der Thiobarbitursäure mit dem Malondialdehyd.

2. Verfahren nach 1., **dadurch gekennzeichnet, dass** die Filterpapierstreifen mit folgender wässrigen Lösung getränkt werden: 0,5 % Thiobarbitursäure, solubilisiert durch 10g % Glykolsäure mit einem pH-Wert von 1,0 bis 3,0. Anschliessend werden die Streifen bei ca. 80°C getrocknet und luft- und lichtgeschützt aufbewahrt.

3. Verfahren nach 1. und 2., **dadurch gekennzeichnet, dass** das Vorhandensein von MDA im Urin durch die Bildung einer roten Korona nachgewiesen wird, deren Intensität von der MDA-Konzentration abhängig ist.

4. Verfahren nach 1. und 2., **dadurch gekennzeichnet, dass** das Vorhandensein von MDA in Lebensmittel- oder kosmetischen Produkten in folgender Form nachgewiesen wird: Entweder als rote Korona im Zentrum des Ölflecks auf dem Papier, oder durch eine typische rosa bis rote Korona, oder durch eine diffuse rötliche Scheibe.
